# EUROPEAN PATENT SPECIFICATION

(11) **EP 4 137 283 B1**
(45) Date of publication and mention of the grant of the patent: **30.04.2025**
(21) Application number: 22190218.2
(22) Date of filing: 12.08.2022
(51) Int. Cl.: B27G 1/00, G06Q 10/04, G06T 7/00, G06T 7/73, G06Q 50/04, B27M 1/08, B27L 5/06, B27L 5/00, G01N 33/46

(54) **A METHOD AND A SYSTEM FOR PROCESSING RAW WOOD LAYERS**
VERFAHREN UND SYSTEM ZUR VERARBEITUNG VON ROHEN HOLZSCHICHTEN
PROCÉDÉ ET SYSTÈME DE TRAITEMENT DE COUCHES DE BOIS BRUT

(30) Priority: 20.08.2021 BE 202105666
(43) Date of publication of application: 22.02.2023
(73) Proprietor: Decospan NV, 8930 Menen (BE)
(72) Inventor: Vanbecelaere, Vincent, 8800 Roeselare (BE); Desmet, Tom, 8560 Gullegem (BE)
(74) Representative: IP HILLS NV

(56) References cited:
- WO-A1-2017/033148
- US-B1- 8 113 098

## Description

### Field of the Invention

The present invention generally relates to the processing of raw wood layers.

### Background of the Invention

Raw wood layers are obtained by slicing or sawing logs of felled trees into thin layers. The aesthetics of the raw wood layer is mainly determined by the used processing method, i.e. the grain and figure, by the presence of defects, and by the tree structure itself. Defects may for example be caused by injuries, insects, or fungi. One example of a raw wood layer is a raw veneer layer. Veneer refers to a thin decorative covering of fine wood applied to a carrier. Raw veneer layers are typically rectangularly shaped and provided in bundles with similar appearance for further processing to an end product.

One type of veneer end products are decorative wood panels for use in e.g. doors, furniture, and wooden floors. Production of such wood panels starts by grading the raw veneer layers. The grading is usually performed by an operator, who manually inspects the grain, figure, and presence of defects for each raw veneer layer. The graded raw veneer layers are then sorted and stored.

In the next step, raw veneer layers of a certain grade are selected to produce a batch of decorative wood panels. Thereto, the raw veneer layers are first trimmed to a fixed length according to the dimensions of the wood panel. The trimmed veneer layers are then clipped in width to exclude imperfections and to straighten the edges. Both trimming and clipping is typically performed by an experienced operator. The clipped veneer layers are then glued adjacent to each other to obtain a veneer sheet. In a further step, the veneer sheets are glued onto a carrier to obtain the decorative wood panel. The aesthetic quality and thereby the yield of the decorative wood panel, is thus determined by the grade of the contained raw veneer layers.

One problem with such panel production is that it contains a lot of manual operations, e.g. during grading, trimming, clipping and adjacent gluing, making the production subjective, relatively unrepeatable, and labour- and time- intensive.

US 8 113 098 B1 discloses a sawmill for machine vision detection of undesirable features in the wood in shingles being cut from billets and automated optimized saw operation. WO 2017/033148A1 discloses a method of optimalization of cutting of flat products from a natural material which has or may have surface and/or internal irregularities or defects, whereby these irregularities are firstly detected, and the cutting plan is devised taking these irregularities into account.

### Summary of the Invention

It is an object of the present invention, amongst others, to solve or alleviate the above identified challenges and problems by improving the production process of decorative wood panels.

According to a first aspect, this object is achieved by a computer implemented method as set out in claim 1.

A quality-determining feature may be any feature of the raw wood layer that can influence it's quality or grade, either negatively or positively. Quality-determining features of the raw wood layer are detected from a digital image of the raw wood layer by an image processing method, such as for example pattern recognition, template matching, or machine learning. Detecting quality-determining features may include, amongst others, recognizing a feature, recognizing the type of a feature, determining the location of a feature, and determining the dimensions of a feature. A candidate cut section is then determined on the raw wood layer surface, i.e. a section of certain shape and size that is to be cut from the raw wood layer later in the production process. In other words, determining a candidate cut section may include determining the shape and dimensions of the candidate cut section. The detected quality-determining features are adjusted such that they only include the features comprised in the candidate cut section. The grade of the candidate cut section, which directly contributes to the yield, is then determined from these adjusted quality-determining features. The grade is thus determined based on how the raw wood layer will be cut later in the manufacturing process, i.e. according to the cut section. This cutting will also contribute to the grade, and will thus also contribute to the yield of the final decorative wood panel, by the removal of undesired quality-determining features.

In addition to the grade, the surface area of the candidate cut section is also taken into account to determine the yield of the raw wood layer. In doing so, a candidate cut section with a substantial surface area and a lower grade can result in a higher yield relative to a candidate cut section with an insignificant surface area and a higher grade. The yield of a candidate cut section may thus be determined more accurately by considering both the surface area and the grade of the candidate cut section. The yield may represent the value of the candidate cut section, i.e. the value potential of the raw wood layer after being cut according to the cut section. The yield may for example be expressed as a numerical score, a letter score, or a monetary value. By iteratively adjusting the candidate cut section and determining the corresponding yield, the candidate cut section that obtains the highest yield from a raw wood layer is determined, i.e. the optimal cut section. In other words, the optimal cut section is determined based on the detected quality-determining features of the raw wood layer. In doing so, a single portion or section that maximizes the yield from a raw wood layer for manufacturing decorative wood panels can be detected.

By determining the optimal cut section, the raw wood layers will be used to their full potential. Further, the cutting losses may be minimized. Trimming and clipping can then be performed according to the determined optimal cut section rather than the assessment of an operator. It is thus an advantage that the production process of decorative wood panels can at least partially be automated, reducing the manual operations during grading, trimming and clipping. This makes the processing of raw wood layers substantially objective and repeatable.

The candidate cut section defines a cropping of the raw wood layer to a rectangular shape. The undesired outer parts of a raw wood layer can then be removed by existing trimming and clipping operations of the raw wood layer according to the edges of the rectangularly shaped candidate cut section.

A minimum length of the candidate cut section is determined based on a length of the decorative wood panels. The candidate cut section may further have a minimum width.

The length of the candidate cut section may for example be selected from a collection of lengths comprising the suitable lengths to manufacture decorative wood panels. The minimum width of the candidate cut section may for example relate to the width of the decorative wood panels, the desired aesthetics of the decorative wood panels, the machinery in the manufacturing process, or the storage capabilities. This can ensure that the processed wood layers meet product requirements and that a suitable search space is defined for the optimization problem, i.e. determining the optimal cut section.

Determining the optimal cut section further comprises adjusting a length, a width, and/or a location of the candidate cut section on the raw wood layer.

The length, width, and/or location of the current candidate cut section may thus be adjusted to improve the grade and/or the surface area of the candidate cut section, and thus the yield of the raw wood layer. This may, for example, be achieved by excluding different or more undesired quality-determining features, by including desired quality-determining features, and/or by adjusting the surface area of the candidate cut section. In other words, adjusting the candidate cut section may include adjusting the shape, dimensions, and/or location of the candidate cut section. By comparing the yield of the adjusted candidate cut section with the current candidate cut section, the optimal cut section with a highest yield can iteratively be determined.

Preferably, the raw wood layer may be a raw veneer layer or a raw wood slat. Raw veneer layers may be obtained by slicing wood logs into thin layers with a thickness of at most 2 mm. Raw wood slats may be obtained by sawing wood logs into slats with a thickness of at least 2 mm.

According to an embodiment, detecting the quality-determining features may be performed by a first classifier trained for detecting said quality-determining features from the digital image.

An algorithm, i.e., the first classifier, may thus be trained by machine learning to detect quality-determining features from a digital image of a raw wood layer. The first classifier may be trained by supervised learning, wherein a plurality of raw wood layer images comprising annotated information on the quality-determining features are presented to the first classifier.

According to an embodiment, the quality-determining features characterize a feature type selected from the group comprising at least one of a defect, a colour, and a cutting pattern.

A defect may comprise an abnormality or irregularity in the raw wood layer. Preferably, a quality-determining feature of the defect type may be selected from the group comprising at least one of a knot, a knot hole, a mineral line, a bird peck, a stain, a burl, a cutting error, a coarse grain, a raised grain, sapwood, fungal damage, an insect defect, a shake, a split, and a twisted fibre.

A cutting pattern may comprise the grain and figure of a raw wood layer, resulting from the used slicing method or sawing method. A quality-determining feature of the cutting pattern type may preferably be selected from the group comprising at least one of a rotary cut, a crown cut, a quarter normal cut, a quarter rift cut, or a combination thereof.

A quality-determining feature of the colour type may preferably be selected from the group comprising at least one of a primary colour, a secondary colour, a colour distribution, a colour difference, and a colour variation.

According to an embodiment, determining the grade of the candidate cut section is performed by a second classifier, trained for determining said grade from the quality-determining features included in said candidate cut section.

An algorithm, i.e., the second classifier, may thus be trained by machine learning to determine the grade, i.e. the quality level, of a candidate cut section on the surface of the raw wood layer from the quality-determining features included in this candidate cut section. In other words, the second classifier only considers the quality-determining features that are located within the boundaries of the candidate cut section to determine the grade, i.e. it ignores the features located outside these boundaries. The second classifier may be trained by supervised learning, wherein a plurality of graded raw wood layers and their respective quality-determining features are presented to the second classifier.

According to an embodiment, at least one production property of the raw wood layer may further be obtained and the second classifier may further be trained for determining said grade from one or more production properties. The grade of the candidate cut section may thus be determined based on the quality-determining features and the one or more production properties.

The one or more production properties may preferably comprise at least one of a raw wood layer width, a raw wood layer length, a raw wood layer thickness, a raw wood layer surface area, a log number, a sequence number, and a quantity of raw wood layers comprised in a bundle.

According to a second aspect, the invention relates to a method for processing a raw wood layer comprising: i) obtaining the raw wood layer; ii) capturing a digital image of the raw wood layer by means of a camera; iii) by a control unit, determining an optimal cut section with a highest yield from said digital image according to the first aspect; iv) sorting the raw wood layer according to the grade of the optimal cut section; v) picking a batch of sorted raw wood layers according to the grade of the optimal cut section; vi) trimming the length of the raw wood layer according to the length of the optimal cut section; and vii) clipping the width of the raw wood layer according to the width of the optimal cut section.

The raw wood layer may for example be obtained by slicing or sawing a log into a substantially thin layer, or by receiving a delivery of sliced or sawn raw wood layers. The raw wood layer may automatically and/or manually be moved in front of a camera such that it can obtain a digital image of said raw wood layer. The wording "in front of the camera" refers to the position wherein an optical lens or assembly of lenses of the camera is able to capture an image of the raw wood layer surface. Determining the optimal cut section with a highest yield may then be performed by a control unit according to the first aspect.

A sorting system may then sort the raw wood layers into batches of the same grade. The sorting system may comprise a manually operated system, an automatically operated system, or a combination thereof. Hereafter, one or more suitable batches of raw wood layers may be selected by a picking system to manufacture a desired decorative panel. The raw wood layers may further be trimmed in length by a trimming system and clipped in width by a clipping system according to the determined optimal cut section. It is thus an advantage that the production process of decorative wood panels may at least partially be automated, reducing the manual operations during grading, trimming and clipping while obtaining the highest yield. This makes the processing of raw wood layers substantially objective and repeatable.

According to a third aspect, the invention relates to a system to process a raw wood layer, the system comprising: i) a camera configured to obtain a digital image of the raw wood layer; ii) a control unit configured to determine an optimal cut section with a highest yield from said digital image according to the first aspect; iii) a sorting system configured to sort the raw wood layer according to the grade of the optimal cut section; iv) a picking system configured to pick a batch of sorted raw wood layers according to the grade of the optimal cut section; v) a trimming system configured to trim the length of the raw wood layer according to the length of the optimal cut section; and vi) a clipping system configured to clip the width of the raw wood layer according to the width of the optimal cut section.

The system is thus configured to obtain a digital image of a raw wood layer. A control unit can then determine the optimal cut section and corresponding grade of the raw wood layer according to the first aspect. A sorting system may then sort and stack the raw wood layers into batches of the same grade. The sorting system may comprise a manually operated system, an automatically operated system, or a combination thereof. Hereafter, a picking system may select one or more suitable batches of raw wood layers to manufacture a desired decorative panel. The raw wood layers may further be trimmed in length by a trimming system and clipped in width by a clipping system according to the determined optimal cut section. It is thus an advantage that the production process of decorative wood panels may at least partially be automated, reducing the manual operations during grading, trimming and clipping while obtaining the highest yield. This makes the processing of raw wood layers substantially objective and repeatable.

According to a fourth aspect, the invention relates to a data processing system configured to perform the computer implemented method according to the first aspect.

According to a fifth aspect, the invention relates to a computer program comprising instructions which, when the program is executed by a computer, cause the computer to perform the computer implemented method according to the first aspect.

According to a sixth aspect, the invention relates to a computer-readable medium comprising instructions which, when executed by a computer, cause the computer to perform the computer implemented method according to the first aspect.

### Brief Description of the Drawings

Fig. 1 shows different processing methods to obtain raw wood layers from a wood log;
Fig. 2 shows a schematic representation of a method for processing a raw wood layer according to an embodiment;
Fig. 3 shows steps according to a computer implemented method for processing a raw wood layer according to an embodiment;
Fig. 4 shows a raw wood layer comprising a rectangularly shaped cut section according to an embodiment;
Fig. 5 shows a training process of a first classifier to detect quality-determining features from a digital image of a raw wood layer according to an embodiment; and
Fig. 6 shows a second classifier trained to determine the grade of a candidate cut section from quality-determining features included in the candidate cut section according to an embodiment.
Fig. 7 shows an example embodiment of a suitable computing system for performing steps according to example aspects of the invention.

### Detailed Description of Embodiment(s)

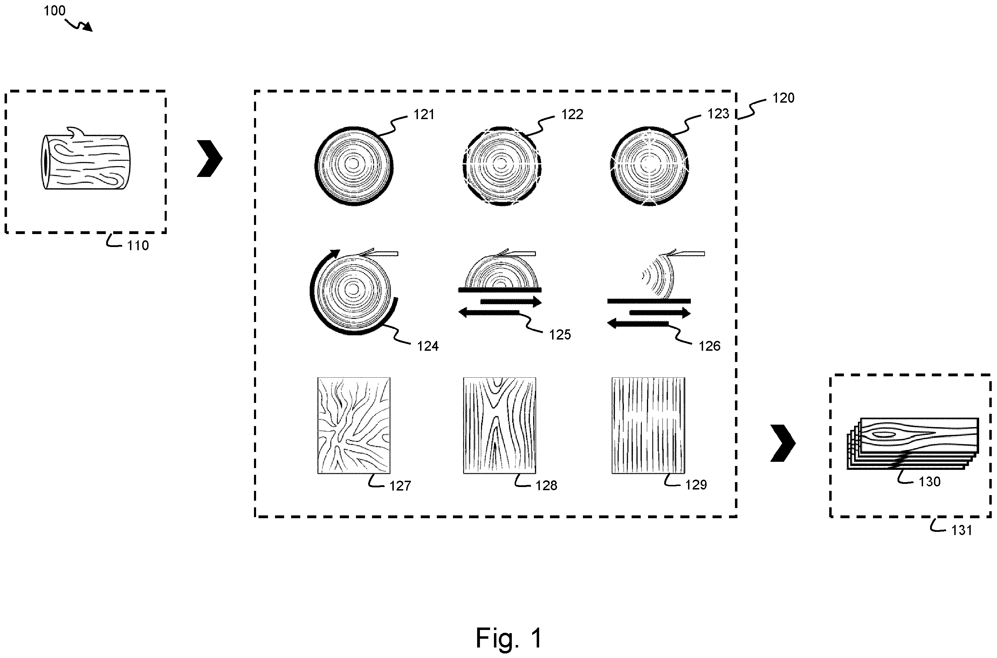

Fig. 1 shows different processing methods 120 to obtain raw wood layers 130 from a wood log 110. The raw wood layers 130 may be raw veneer layers or raw wood slats, depending on the thickness of the layers and the used processing method 120 to obtain them. Raw veneer layers may be obtained by slicing a wood log 110 into thin layers with a thickness of at most 2 mm by means of a blade, e.g. a peeling knife or a slicing knife. Raw wood slats may be obtained by sawing a wood log 110 into slats with a thickness of at least 2 mm by means of a sawblade. A plurality of raw wood layers 130 can be obtained from a single log 110. The yield of raw wood layers 130 and their aesthetics, i.e. the grain and figure, is substantially determined by the used processing method 120.

Fig. 1 further shows examples of commonly used processing methods 120 for raw veneer layers, i.e. slicing methods, such as rotary cutting 121, crown cutting 122, and quarter normal cutting 123. In rotary cutting 121, the log is turned around its axis 124 against a blade that peels- or shaves off one continuous or semi-continuous raw veneer layer. Rotary cut raw veneer layers 127 may have a broad grain pattern which is typically less desirable for applications where aesthetics are important. In crown cutting 122, the log is moved back and forth 125 against a blade across the growth rings of the wood log 110, thereby cutting through the heart of the log 110. Crown cut raw veneer layers 128 may have a flame grain pattern that is substantially similar to the grain pattern of plain sawn wood. In quarter normal cutting 123, the log is sliced substantially perpendicular to its growth rings 126, thereby resulting in quarter normal cut raw veneer layers 129 with a straight-grained pattern. Alternatively, a wood log 110 can be sawn according to different sawing methods, e.g. plain sawn, quarter sawn, or rift sawn, to obtain the desired aesthetics and/or yield of raw wood slats.

The obtained raw wood layers 130 may further be grouped into bundles 131, e.g. a collection of layers obtained from the same log 110 or having a substantially similar appearance, before being further processed to an end product such as for example decorative wood panels. Decorative wood panels may for example be used for, amongst others, wooden doors, wooden furniture, wooden floors, or wooden wall cladding. The bundle 131 of raw wood layers 130 may further be labelled with an information carrier that comprises at least one production property of the bundle 131, e.g. a raw wood layer width, a raw wood layer length, a raw wood layer thickness, a raw wood layer surface area, a log number, a sequence number, or a quantity of raw wood layers comprised in a bundle. In other words, the information carrier may comprise product information on the bundle 131 as a whole and/or on the individual raw wood layers 130 included in said bundle 131. Such an information carrier may for example be, amongst others, a barcode, a QR-code, an RFID tag, or any other information carrier known to the person skilled in the art.

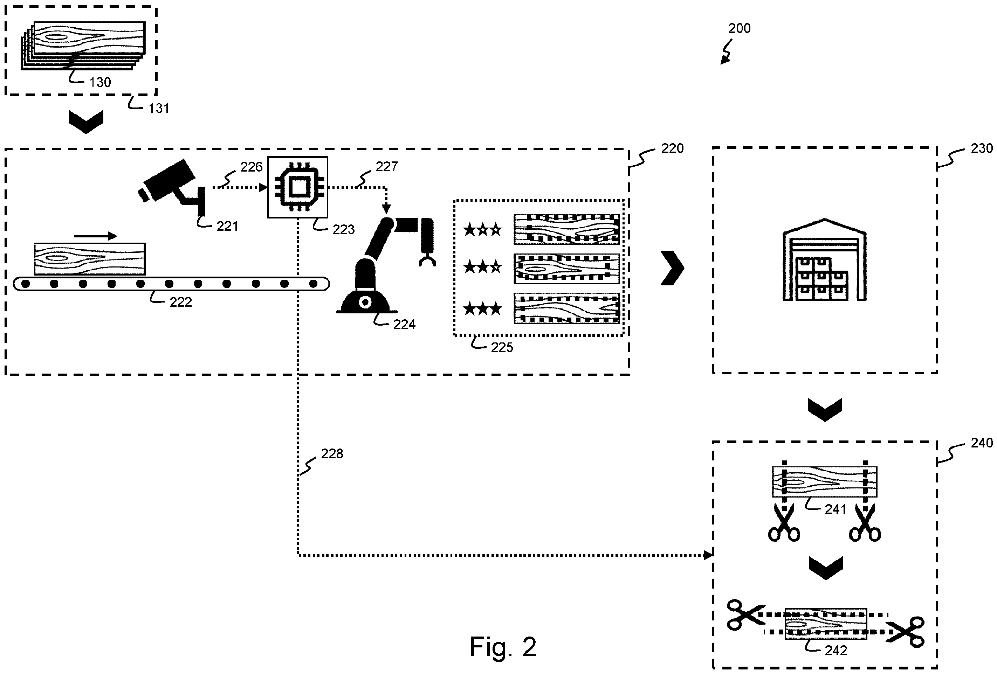

Fig. 2 shows a schematic representation of a method 200 for processing a raw wood layer according to an embodiment. Method 200 may be part of a manufacturing process that produces end products such as decorative wood panels from raw wood layers 130. In a first step, a single raw wood layer 130 or a bundle 131 of raw wood layers is obtained, e.g. from a preceding production step such as illustrated by Fig. 1 , or by receiving a delivery of such sliced or sawn raw wood layers. The product information of the single raw wood layer 130 or bundle 131 of raw wood layers may further be obtained by scanning or reading out the information carrier.

In a following step, the raw wood layer 130 is moved in front of a camera 221 that takes a digital image of the raw wood layer 130. The wording "in front of the camera" refers to the position wherein an optical lens or assembly of lenses of the camera 221 is able to capture an image of the raw wood layer's 130 surface. Preferably, a bundle 131 of raw wood layers is moved in front of the camera 221 such that a digital image can be obtained of an outer raw wood layer of the bundle 131, i.e., the layer of the bundle 131 that is facing the camera 221. The camera 221 may for example be, amongst others, a rotating line camera, a line-scan camera, a 3D camera, an IP camera, a high-speed camera, a machine vision camera, a spectral camera, a contact image sensor, an area scan camera, a smart camera, or any other camera known to the person skilled in the art. The raw wood layer 130 or bundle 131 may be moved manually in front of the camera 221. Preferably, the one or more layers are moved automatically or semi-automatically in front of the camera 221, e.g. by means of a conveyor belt 222.

The captured digital image of the raw wood layer 130 is then received 226 by a control unit 223 that is configured to determine an optimal cut section, i.e. a candidate cut section of certain shape and size that is to be cut from the raw wood layer 130 later in the production process such that the yield from said raw wood layer 130 is optimal and thus highest. The yield may represent the value of a candidate cut section, i.e. the value potential of the raw wood layer after being cut according to the candidate cut section. The yield may for example be expressed as a numerical score, a letter score, or a monetary value.

Hereto, the control unit 223 is configured to detect quality-determining features of the raw wood layer 130 from the received digital image, e.g. wood grain, wood figure, wood defects, and colours. A quality-determining feature may thus have a negative effect on the grade or quality of a candidate cut section, e.g. a wood defect, or a positive effect on the grade or quality of a candidate cut section, e.g. a desired wood grain or figure. A candidate cut section can then be determined on the raw wood layer's 130 surface. The candidate cut section is a single section or portion of the raw wood layer that has a certain shape and size, i.e. certain dimensions. Based on the detected features that are included in the candidate cut section, the control unit 223 can determine a grade of the candidate cut section, i.e. a quality-level. The raw wood layer 130 is thus graded based on how the raw wood layer 130 will be cut later in the manufacturing process, i.e. according to the cut section. The determined grade for a raw wood layer thus includes the contribution of this cutting, e.g. by the removal of undesired quality-determining features. The grade contributes to the yield of a raw wood layer 130 and will thus also contribute to the yield of the final end product, e.g. a decorative wood panel. In addition to the grade, the surface area of the candidate cut section is also taken into account to determine the yield of the raw wood layer 130. In doing so, a candidate cut section with a substantial surface area and a lower grade can result in a higher yield relative to a candidate cut section with an insignificant surface area and a higher grade.

The determined grade and the surface area of the candidate cut section are then further used to determine the yield of the candidate cut section. By iteratively adjusting the candidate cut section and determining the corresponding yield, the control unit 223 can determine the candidate cut section that obtains the highest yield from a raw wood layer 130, i.e. the optimal cut section. In other words, the optimal cut section is determined based on the detected quality-determining features of the raw wood layer 130. Considering both the surface area and the grade of a candidate cut section may improve the determining of the optimal cut section as the yield is determined more accurately.

In a next step, the graded raw wood layers 130 can be sorted and stacked into batches 225 of the same grade by means of a sorting system 224. Hereto, the control unit 223 may transmit 227 the determined grade and optimal cut section of a raw wood layer 130 or bundle 131 to sorting system 224. The sorting system 224 may comprise a manually operated system, an automatically operated system, or a combination thereof. Such a sorting system may for example be, amongst others, a robotic arm, a veneer stacking machine, a pusher sorter, a cross belt sorter, or any other sorting system known to the person skilled in the art.

The graded raw wood layer 130 or bundle 131 may further be labelled with the determined grade and/or the optimal cut section, for example by storing them on an information carrier belonging to the graded raw wood layer 130 or bundle 131. The stored information related to the optimal cut section may for example include coordinates of the optimal cut section, dimensions of the optimal cut section, the position of the optimal cut section, or computer numerical control-, CNC-, instructions for cutting the optimal cut section. Alternatively, this labelling of the graded raw wood layer 130 or bundle 131 may be achieved by marking, e.g. painting, drawing, printing, or engraving, the optimal cut section and/or the determined grade on the raw wood layer's surface. By labelling the graded layers, the required data on the determined grade and the optimal cut section may be transmitted to the further steps 230, 240 in the processing method 200 of a raw wood layer 130.

In a following step, the sorted batches 225 of graded raw wood layers 130 or bundles 131 may be transported to a storage space 230, where they can be stored until they are needed for manufacturing an end product such as decorative wood panels. One or more suitable batches 225, i.e. batches 225 of raw wood layers 130 or bundles 131 with a desired grade, may be selected and picked from the storage space 230 by a picking system to be further processed into decorative wood panels. The batches 255 may for example be picked according to the desired quality and dimensions of the decorative wood panels. The picked batches 225 are then transported to a cutting system for cutting 240 the raw wood layers 130 according to their determined optimal cut section. Alternatively, the sorted batches 225 of graded raw wood layers 130 or bundles 131 may be transported substantially directly to the cutting system, i.e. without being stored in a storage space 230.

Cutting 240 the raw wood layers 130 according to their determined optimal cut section may further comprise a trimming step 241 and a clipping step 242. In the trimming step 241, the raw wood layers 130 are trimmed according to the length of the optimal cut section by means of a trimming system. Hereafter, the raw wood layers 130 are clipped to the width of the optimal cut section by a clipping system, during the clipping step 242. Trimming 241 and clipping 242 may for example be performed by means of, amongst others, a blade, a knife, a sawblade, laser cutting, waterjet cutting, machine cutting, or any other cutting means known to the person skilled in the art. Alternatively, trimming 241 and clipping 242 may be performed in the reversed order, performed in a single step, or performed by a single apparatus or system.

Trimming 241 and clipping 242 may be performed manually or semi-automatically by an operator. The operator may for example align the optimal cut section of the raw wood layers 130 with the cutting means of the trimming system or clipping system, such that the layers 130 are cut according to the determined optimal cut section. Hereto, the optimal cut section may have been transmitted 228 from the control unit 223 to the cutting step 240 by for example marking the surface of the raw wood layers 130, e.g. by painting, drawing, printing, or engraving the optimal cut section on the raw wood layer's surface. Alternatively, an information carrier may be scanned or read out prior to trimming 241 or clipping 242. The optimal cut section may then for example be conveyed to the operator by projecting the optimal cut section on the raw wood layer's surface, e.g. by a light-source or a laser.

Alternatively, trimming 241 and clipping 242 may further be performed automatically, i.e. without substantial interference of an operator. An operator may for example feed the raw wood layers 130 of a picked batch 225 into an automated cutting system 240, configured to automatically trim 241 and clip 242 the raw wood layers according to their optimal cut section. Alternatively, feeding the raw wood layers 130 of a picked batch 225 into the automated cutting system 240 may further be performed without substantial interference of an operator, e.g. by means of a robotic arm or a conveyor belt.

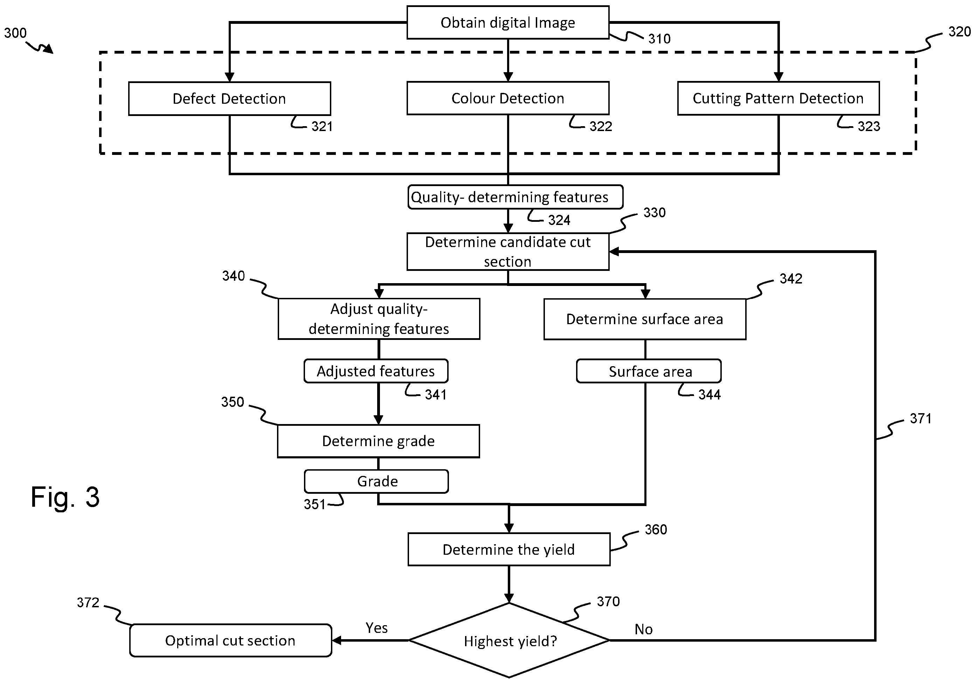

Fig. 3 illustrates steps 300 performed for determining the optimal cut section with an optimal yield according to an example embodiment. According to example embodiments, steps 300 may be performed by control unit 223 to determine the optimal cut section of raw wood layers 130. A digital image of a raw wood layer is obtained in a first step 310, to detect the quality-determining features 324 of said raw wood layer by means of an image processing method in a second step 320. Such an image processing method may for example be, amongst others, pattern recognition, template matching, machine learning, or any other image processing method known to the person skilled in the art. Detecting quality-determining features 324 may include, amongst others, recognizing a feature, recognizing the type of a feature, determining the location of a feature on the raw wood layer, and determining the dimensions of a feature. The type of detected quality-determining feature 324 may preferably be a defect, a colour, or a cutting pattern. A quality-determining feature may thus have a negative or a positive effect on the grade of a candidate cut section.

A defect is to be understood as an abnormality or irregularity in the raw wood layer, for example caused by injuries, insects, or fungi. Preferably, a quality-determining feature 324 of the defect type may be a knot, a knot hole, a mineral line, a bird peck, a stain, a burl, a cutting error, a coarse grain, a raised grain, sapwood, fungal damage, an insect defect, a shake, a split, or a twisted fibre.

A cutting pattern is to be understood as the grain and figure of a raw wood layer resulting from the used slicing method or sawing method to obtain the raw wood layer from a wooden log. A quality-determining feature 324 of the cutting pattern type may preferably be a rotary cut, a crown cut, a quarter normal cut, a quarter rift cut, or a combination thereof. A raw wood layer with a certain cutting pattern type may include variations in the grain and figure that contribute to the grade, which may further be detected as a quality-determining feature 324.

A quality-determining feature of the colour type may preferably be a primary colour, a secondary colour, a colour distribution, a colour difference, or a colour variation. A colour difference may for example be the difference between the primary colour and the secondary colour. A colour variation may for example be the variation between the primary colour and a reference colour.

The image processing step 320 may further be performed by a single algorithm configured to detect one or more of these feature types, i.e. defects, colours, and cutting patterns. Alternatively, the image processing step 320 may comprise one or more different algorithms specifically suited for defect detection 321, colour detection 322, and cutting pattern detection 323.

In a following step 330, the detected quality-determining features 324 are used to determine a candidate cut section on the raw wood layer surface, i.e. a section of certain shape and size that is to be cut from the raw wood layer later in the production process. In other words, determining a candidate cut section may include determining the shape and dimensions of the candidate cut section. Preferably, the candidate cut section defines a cropping of the raw wood layer to a substantially rectangular shape. The term 'cropping' is to be understood as that the outside edges of the rectangularly shaped candidate cut section are substantially parallel to the respective outer edges of the raw wood layer from which the candidate cut section is to be cut. In other words, the rectangularly shaped cut section may be substantially parallel to the longitudinal direction of the raw wood layer.

A minimum length of the candidate cut section may further be determined based on a length of the decorative wood panels, i.e. an end product to be produced from the raw wood layer. For example, the length of the candidate cut section may be selected from a collection of lengths representative for all suitable lengths to produce decorative wood panels.

The candidate cut section may further be characterized by a minimum width. The minimum width of the candidate cut section may for example, amongst others, relate to the width of the decorative wood panels, the desired aesthetics of the decorative wood panels, the machinery in the manufacturing process, or the storage capabilities. This can ensure that the processed wood layers meet product requirements and that a suitable search space is defined for the optimization problem, i.e. determining the optimal cut section.

In the next step 340, the detected quality-determining features 324 are adjusted to only include the features 341 comprised in the candidate cut section, i.e. the adjusted features 341. In other words, detected quality-determining features 324 that are not spatially located within the boundaries of the candidate cut section are not considered to determine the grade.

The grade 351 of the candidate cut section, which contributes to the yield, is then determined from these adjusted quality-determining features 341 during a grading step 350. The grade 351 is thus determined based on how the raw wood layer will be cut later in the manufacturing process, i.e. according to the candidate cut section. This cutting will contribute to the grade 351, and will thus also contribute to the yield of the final decorative wood panel, by the removal of undesired quality-determining features. In other words, when the raw wood layer is cut according to the candidate cut section later in the manufacturing process, the detected features 324 that are not spatially located within the boundaries of the candidate cut section would be permanently removed from the wood layer, thereby no longer contributing to the grade 351 of the processed wood layer.

In addition to the grade 351, the surface area 344 of the candidate cut section is also taken into account to determine the yield 360 of the raw wood layer. In doing so, a candidate cut section with a substantial surface area 344 and a lower grade 351 can result in a higher yield relative to a candidate cut section with an insignificant surface area 344 and a higher grade 351. The yield may represent the value of the candidate cut section, i.e. the value potential of the raw wood layer after being cut according to the cut section. The yield may for example be expressed as a numerical score, a letter score, or a monetary value.

The length, width, and/or location of the current candidate cut section may then iteratively be adjusted 371 to improve the grade 351 and/or the surface area 344 of the candidate cut section, and thus the yield of the raw wood layer. This may for example be achieved by excluding different or more undesired quality-determining features 324, by including desired quality-determining features, or by adjusting the surface area 344 of the candidate cut section. By comparing 370 the yield of the adjusted candidate cut section with the current candidate cut section, the optimal cut section 372 with a highest yield, i.e. the optimal yield, can iteratively be determined.

As such, iteratively adjusting 371 the candidate cut section and determining the corresponding yield 360 allows determining the candidate cut section that results in the highest yield from a raw wood layer, i.e. the optimal cut section 372. In other words, the optimal cut section 372 is determined based on the detected quality-determining features 324 of the raw wood layer. This allows to identify a single portion or section that maximizes the yield from a raw wood layer for manufacturing decorative wood panels.

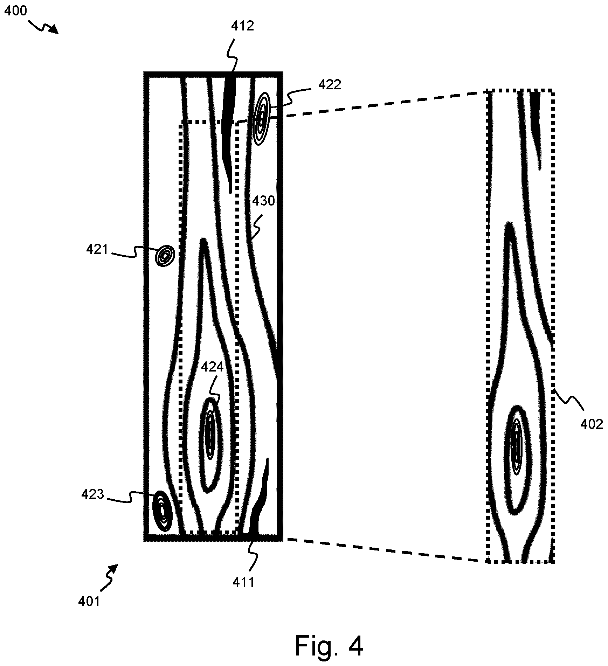

Fig. 4 shows a raw wood layer 401 comprising a rectangularly shaped cut section 402 according to an embodiment. The raw wood layer 401 comprises a plurality of quality-determining features of the defect type, including two lengthwise cracks or separations of the wood, i.e. splits 411, 412, and four branches that were removed from the tree, i.e. knots 421, 422, 423, 424. Raw wood layer 401 further comprises a substantial flame grain pattern 430, characteristic for a quality-determining feature of the cutting pattern type, i.e. a crown cut. The rectangularly shaped cut section 402 is thus determined to obtain the highest yield from raw wood layer 401 by for example excluding undesired quality-determining features 411, 421, 422, 423 and including the most desirable section of the flame grain pattern, while also ensuring a substantial surface area.

The image processing method, configured to detect the quality-determining features 411, 412, 421, 422, 423, 424, 430 of raw wood layer 401, may preferably be a first classifier trained for detecting said quality-determining features from the digital image of raw wood layer 401. An algorithm, i.e., the first classifier, may thus be trained by machine learning to detect quality-determining features from a digital image of a raw wood layer. The trained classifier may be, amongst others, an artificial neural network, a residual neural network, a decision tree, a support-vector machine, regression analysis, K-means clustering, a Bayesian network, a genetic algorithm, or any other classifier known to the person skilled in the art. The first classifier may be trained by supervised learning, wherein a plurality of raw wood layer images comprising annotated information on the quality-determining features are presented to the first classifier.

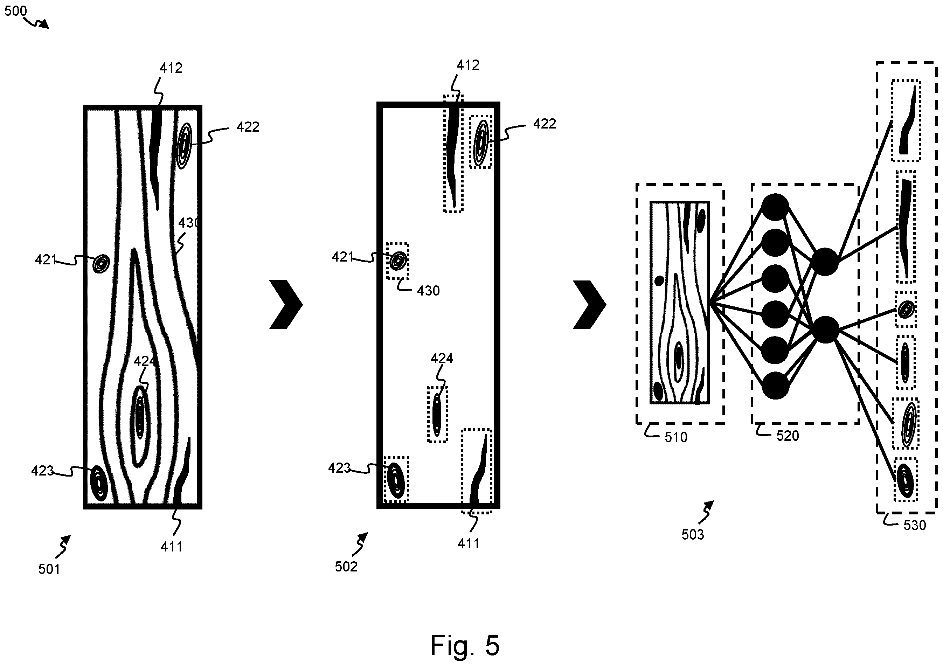

Fig. 5 shows a training process 500 of a first classifier 520 to detect quality-determining features 530 from the digital image 510 of a raw wood layer according to an embodiment. The training process 500 may start by gathering a plurality of digital images 501 of raw wood layers, i.e. training data. In a next step 502, the digital images 501 of raw wood layers may be annotated with information on the quality-determining features 411, 412, 421, 422, 423, 424 that should be detected by the first classifier, e.g. by a graphical image annotation tool. In this manner, a dataset of annotated images is constructed that comprises, amongst others, the location of the features, the type of features, the boundaries of the features, and the dimensions of the features. The training dataset may include at least 5000 annotations, preferably at least 10 000, even more preferably at least 30 000. Alternatively or complementary, the first classifier may be pre-trained on a benchmark dataset, e.g. the COCO dataset, the open images dataset, the imageNet dataset, or the CIFAR-10 dataset. The first classifier may further be trained by unsupervised learning or by reinforcement learning.

Training the first classifier 520 by machine learning can thus result in a machine learning model 503 that can detect quality-determining features 530 of a raw wood layer from a digital image 510 of the layer. The first classifier 520 may comprise a single classifier trained to detect one or more types of quality-determining features 530, i.e. defects, colours, and cutting patterns. Alternatively, the first classifier 520 may comprise one or more classifiers trained to detect a specific type of quality-determining features 530, e.g. for defect detection, for colour detection, and for cutting pattern detection.

A trained classifier for colour detection may detect the colour of the raw wood layer's surface at distinct spatial points on the digital image. Preferably, the colour is determined in a perceptually uniform colour space such as the CIELAB colour space or CIELUV colour space. In doing so, a numerical change in colour value can correspond relatively better to a similar colour change as perceived by the human eye, compared to an additive or subtractive colour model such as RGB, RYB, CMY, or CMYK. Additionally, it allows to use the CIEDE2000 formula to quantify the distance or difference between detected colour values, or between a detected colour value and a reference colour value, approximating how humans perceive colour differences.

Determining the grade of the candidate cut section may preferably be performed by a second classifier trained for determining the grade from the quality-determining features included in said candidate cut section, i.e. the adjusted features. An algorithm, i.e., the second classifier, may thus be trained by machine learning to determine the grade, i.e. the quality level, of a candidate cut section on the surface of the raw wood layer from the quality-determining features included in this candidate cut section. In other words, the second classifier only considers the quality-determining features that are located within the boundaries of the candidate cut section to determine the grade, i.e. it ignores the features located outside these boundaries.

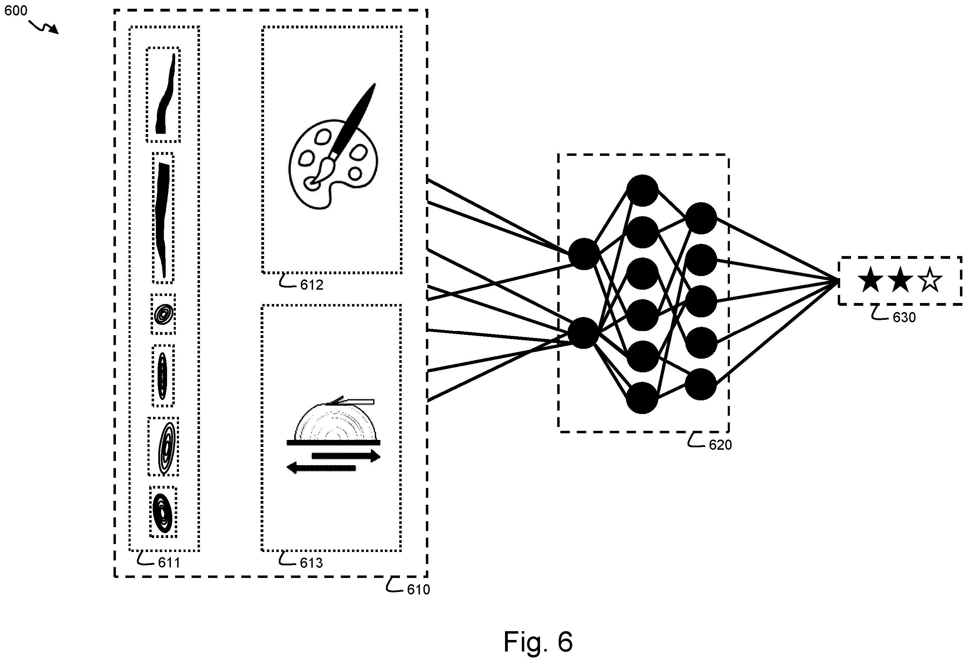

Fig. 6 shows a second classifier 620 trained to determine the grade 630 of a candidate cut section from the quality-determining features 610 included in the candidate cut section according to an embodiment. The quality-determining features 610 may for example be one or more of the defect type 611, the colour type 612, and the cutting pattern type 613. Preferably, these quality-determining features 610 have been detected by the first classifier, i.e. the input of the second classifier may be the output of the first classifier.

The second classifier 620 may further be trained for determining the grade 630 based on one or more production properties of the raw wood layer or bundle of raw wood layers. The grade 630 of the candidate cut section may thus be determined based on the quality-determining features 610 and one or more production properties of the raw wood layer. These production properties may for example be obtained by scanning or reading out the information carrier of the raw wood layer or bundle of layers, e.g. a barcode, a QR-code, or an RFID tag. A production property that contributes to determining the grade 630 of the candidate cut section may for example be, amongst others, a raw wood layer width, a raw wood layer length, a raw wood layer thickness, a raw wood layer surface area, a log number, a sequence number, or a quantity of raw wood layers comprised in a bundle.

The second classifier 620 may be trained by supervised learning, wherein the grade of raw wood layers, their respective quality-determining features 610, and their production properties are presented to the second classifier 620. In other words, a training dataset is constructed that relates the grade 630 of a raw wood layer to the quality-determining features 610 and the production properties of that layer. Alternatively or complementary, the second classifier 620 may be pre-trained on a benchmark dataset, trained by unsupervised learning, or trained by reinforcement learning. The trained classifier may, amongst others, be an artificial neural network, a decision tree, a support-vector machine, regression analysis, a Bayesian network, a genetic algorithm, or any other classifier known to the person skilled in the art.

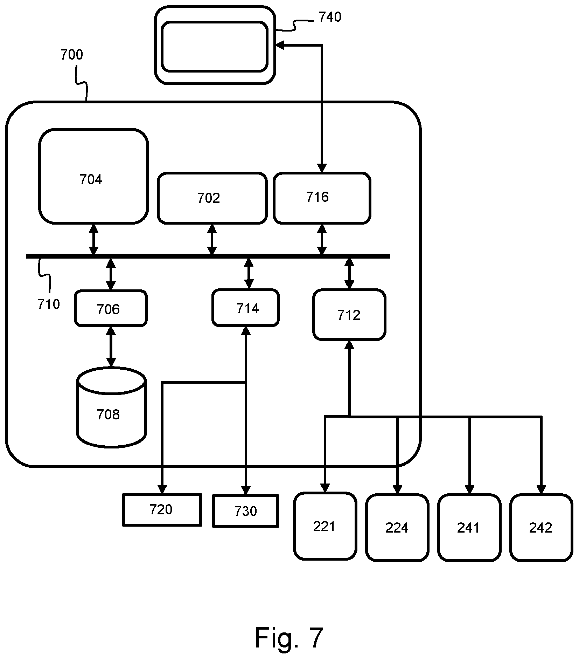

Fig. 7 shows a suitable computing system 700 enabling to implement embodiments of the above described method according to the invention. Computing system 700 may in general be formed as a suitable general-purpose computer and comprise a bus 710, a processor 702, a local memory 704, one or more optional input interfaces 714, one or more optional output interfaces 716, a communication interface 712, a storage element interface 706, and one or more storage elements 708. Bus 710 may comprise one or more conductors that permit communication among the components of the computing system 700. Processor 702 may include any type of conventional processor or microprocessor that interprets and executes programming instructions. Local memory 704 may include a random-access memory (RAM) or another type of dynamic storage device that stores information and instructions for execution by processor 702 and/or a read only memory (ROM) or another type of static storage device that stores static information and instructions for use by processor 702. Input interface 714 may comprise one or more conventional mechanisms that permit an operator or user to input information to the computing device 700, such as a keyboard 720, a mouse 730, a pen, voice recognition and/or biometric mechanisms, a camera, etc. Output interface 716 may comprise one or more conventional mechanisms that output information to the operator or user, such as a display 740, etc. Communication interface 712 may comprise any transceiver-like mechanism such as for example one or more Ethernet interfaces that enables computing system 700 to communicate with other devices and/or systems such as for example, amongst others, a camera 221, a sorting system 224, a trimming system 241, or a clipping system 242. The communication interface 712 of computing system 700 may be connected to such another computing system by means of a local area network (LAN) or a wide area network (WAN) such as for example the internet. Storage element interface 706 may comprise a storage interface such as for example a Serial Advanced Technology Attachment (SATA) interface or a Small Computer System Interface (SCSI) for connecting bus 710 to one or more storage elements 708, such as one or more local disks, for example SATA disk drives, and control the reading and writing of data to and/or from these storage elements 708. Although the storage element(s) 708 above is/are described as a local disk, in general any other suitable computer-readable media such as a removable magnetic disk, optical storage media such as a CD or DVD, - ROM disk, solid state drives, flash memory cards, etc. could be used.

Although the present disclosure has been illustrated by reference to specific embodiments, it will be apparent to those skilled in the art that the invention is not limited to the details of the foregoing illustrative embodiments. The invention is limited by the appended claims. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A computer implemented method (300) for processing a raw wood layer for the manufacturing of decorative wood panels, the method comprising the following steps:
- obtaining (310) a digital image of the raw wood layer;
- detecting (320) quality-determining features (324) of the raw wood layer from the digital image;
wherein the computer-implemented method is **characterized by**
- determining (330) a candidate cut section on the raw wood layer, wherein the candidate cut section defines a cropping of the raw wood layer to a rectangular shape (402) having a minimum length that is determined based on a length of the decorative wood panels;
- determining (350) a grade of the candidate cut section from the quality-determining features included in said cut section;
- determining (360) a yield of the candidate cut section based on an area (344) of the candidate cut section and the grade (351); and
- determining (370) an optimal cut section as the candidate cut section that results in a highest yield by, iteratively:
- adjusting a length, a width, and/or a location of the candidate cut section (402) on the raw wood layer (401); and
- determining the yield for the adjusted candidate cut section (330, 350, 360).

2. The computer implemented method according to claim 1, wherein detecting (320) the quality-determining features is performed by a first classifier (520) trained for detecting said quality-determining features (530) from the digital image (510).

3. The computer implemented method according to any of the preceding claims, wherein the quality-determining features (324) characterize a feature type selected from the group comprising at least one of a defect (321), a colour (322), and a cutting pattern (323).

4. The computer implemented method according to claim 3, wherein a quality-determining feature of the defect type (321) is selected from the group comprising at least one of a knot, a knot hole, a mineral line, a bird peck, a stain, a burl, a cutting error, a coarse grain, a raised grain, sapwood, fungal damage, an insect defect, a shake, a split, and a twisted fibre.

5. The computer implemented method according to claim 3, wherein a quality-determining feature of the cutting pattern type (323) is selected from the group comprising at least one of a rotary cut, a crown cut, a quarter normal cut, a quarter rift cut, or a combination thereof.

6. The computer implemented method according to claim 3, wherein a quality-determining feature of the colour type (322) is selected from the group comprising at least one of a primary colour, a secondary colour, a colour distribution, a colour difference, and a colour variation.

7. The computer implemented method according to any of the preceding claims, wherein determining (350) the grade of the candidate cut section is performed by a second classifier (620), trained for determining said grade (630) from the quality-determining features (610) included in said candidate cut section.

8. The computer implemented method according to claim 7, further comprising obtaining at least one production property of the raw wood layer and wherein the second classifier (620) is further trained for determining said grade (630) from one or more production properties.

9. A method (200) for processing a raw wood layer (130) comprising:
- obtaining the raw wood layer (130);
- capturing a digital image of the raw wood layer by means of a camera (221);
- by a control unit (223), determining an optimal cut section with a highest yield from said digital image by performing the computer-implemented method according to any one of claims 1 to 8;
- sorting (225) the raw wood layer according to the grade of the optimal cut section;
- picking a batch of sorted raw wood layers according to the grade of the optimal cut section;
- trimming (241) the length of the raw wood layer according to the length of the optimal cut section; and
- clipping (242) the width of the raw wood layer according to the width of the optimal cut section.

10. A system to process a raw wood layer, the system comprising:
- a camera (221) configured to obtain a digital image of the raw wood layer (130);
- a control unit (223) configured to determine an optimal cut section with a highest yield from said digital image by performing the computer-implemented method according to any one of claims 1 to 8;
- a sorting system (224) configured to sort (225) the raw wood layer according to the grade of the optimal cut section;
- a picking system configured to pick a batch of sorted raw wood layers according to the grade of the optimal cut section;
- a trimming system (241) configured to trim the length of the raw wood layer according to the length of the optimal cut section; and
- a clipping system (242) configured to clip the width of the raw wood layer according to the width of the optimal cut section.

11. A data processing system configured to perform the computer implemented method (300) according to any one of claims 1 to 8.

12. A computer program comprising instructions which, when the program is executed by a computer, cause the computer to perform the computer implemented method (300) according to any one of claims 1 to 8.

13. A computer-readable medium comprising instructions which, when executed by a computer, cause the computer to perform the computer implemented method (300) according to any one of claims 1 to 8.

## Patentansprüche

1. Computerimplementiertes Verfahren (300) zum Verarbeiten einer rohen Holzschicht für die Herstellung von dekorativen Holzplatten, wobei das Verfahren die folgenden Schritte umfasst:
- Erhalten (310) eines digitalen Bildes der rohen Holzschicht;
- Erfassen (320) von Qualitätsbestimmungsmerkmalen (324) der rohen Holzschicht aus dem digitalen Bild;
wobei das computerimplementierte Verfahren **gekennzeichnet ist durch**
- Bestimmen (330) eines Kandidatenschnittabschnittes auf der rohen Holzschicht, wobei der Kandidatenschnittabschnitt ein Zuschneiden der rohen Holzschicht zu einer rechteckigen Form (402) mit einer minimalen Länge definiert, die basierend auf einer Länge der dekorativen Holzplatten bestimmt wird;
- Bestimmen (350) eines Grads des Kandidatenschnittabschnittes aus den Qualitätsbestimmungsmerkmalen, die in dem Schnittabschnitt enthalten sind;
- Bestimmen (360) einer Ausbeute des Kandidatenschnittabschnittes basierend auf einer Fläche (344) des Kandidatenschnittabschnittes und dem Grad (351); und
- Bestimmen (370) eines optimalen Schnittabschnittes als den Kandidatenschnittabschnitt, der in einer höchsten Ausbeute resultiert, durch, auf iterative Weise:
- Einstellen einer Länge, einer Breite und/oder einer Stelle des Kandidatenschnittabschnittes (402) auf der rohen Holzschicht (401); und
- Bestimmen der Ausbeute für den eingestellten Kandidatenschnittabschnitt (330, 350, 360).

2. Computerimplementiertes Verfahren nach Anspruch 1, wobei das Erfassen (320) der Qualitätsbestimmungsmerkmale durch einen ersten Klassifizierer (520) durchgeführt wird, der zum Erfassen der Qualitätsbestimmungsmerkmale (530) aus dem digitalen Bild (510) trainiert ist.

3. Computerimplementiertes Verfahren nach einem der vorhergehenden Ansprüche, wobei die Qualitätsbestimmungsmerkmale (324) einen Merkmalstyp kennzeichnen, der aus der Gruppe ausgewählt ist, die zumindest eines von einem Defekt (321), einer Farbe (322) und einem Schnittmuster (323) umfasst.

4. Computerimplementiertes Verfahren nach Anspruch 3, wobei ein Qualitätsbestimmungsmerkmal des Defekttyps (321) aus der Gruppe ausgewählt ist, die zumindest eines von einem Knoten, einem Knotenloch, einer Minerallinie, einem Vogelpick, einem Fleck, einer Noppe, einem Schneidfehler, einem groben Korn, einem erhabenen Korn, Splintholz, Pilzschäden, einem Insektendefekt, einem Schütteln, einem Spalt und einer verdrehten Faser umfasst.

5. Computerimplementiertes Verfahren nach Anspruch 3, wobei ein Qualitätsbestimmungsmerkmal des Schnittmustertyps (323) aus der Gruppe ausgewählt ist, die zumindest eines von einem Rotationsschnitt, einem Kronenschnitt, einem Viertelnormalschnitt, einem Viertelrissschnitt oder einer Kombination davon umfasst.

6. Computerimplementiertes Verfahren nach Anspruch 3, wobei ein Qualitätsbestimmungsmerkmal des Farbtyps (322) aus der Gruppe ausgewählt ist, die zumindest eines von einer Primärfarbe, einer Sekundärfarbe, einer Farbverteilung, einem Farbunterschied und einer Farbvariation umfasst.

7. Computerimplementiertes Verfahren nach einem der vorhergehenden Ansprüche, wobei das Bestimmen (350) des Grads des Kandidatenschnittabschnittes durch einen zweiten Klassifizierer (620) durchgeführt wird, der zum Bestimmen des Grads (630) aus den Qualitätsbestimmungsmerkmalen (610), die in dem Kandidatenschnittabschnitt enthalten sind, trainiert ist.

8. Computerimplementiertes Verfahren nach Anspruch 7, ferner umfassend Erhalten von zumindest einer Produktionseigenschaft der rohen Holzschicht und wobei der zweite Klassifizierer (620) ferner zum Bestimmen des Grads (630) aus einer oder mehreren Produktionseigenschaften trainiert ist.

9. Verfahren (200) zum Verarbeiten einer rohen Holzschicht (130), umfassend:
- Erhalten der rohen Holzschicht (130);
- Aufnehmen eines digitalen Bildes der rohen Holzschicht mittels einer Kamera (221);
- Bestimmen, durch eine Steuereinheit (223), eines optimalen Schnittabschnittes mit einer höchsten Ausbeute aus dem digitalen Bild durch Durchführen des computerimplementierten Verfahrens nach einem der Ansprüche 1 bis 8;
- Sortieren (225) der rohen Holzschicht gemäß dem Grad des optimalen Schnittabschnittes;
- Auswählen einer Charge von sortierten rohen Holzschichten gemäß dem Grad des optimalen Schnittabschnittes;
- Beschneiden (241) der Länge der rohen Holzschicht gemäß der Länge des optimalen Schnittabschnittes; und
- Stutzen (242) der Breite der rohen Holzschicht gemäß der Breite des optimalen Schnittabschnittes.

10. System zum Verarbeiten einer rohen Holzschicht, wobei das System Folgendes umfasst:
- eine Kamera (221), die konfiguriert ist, um ein digitales Bild der rohen Holzschicht (130) zu erhalten;
- eine Steuereinheit (223), die konfiguriert ist, um einen optimalen Schnittabschnitt mit einer höchsten Ausbeute aus dem digitalen Bild durch Durchführen des computerimplementierten Verfahrens nach einem der Ansprüche 1 bis 8 zu bestimmen;
- ein Sortiersystem (224), das konfiguriert ist, um die rohe Holzschicht gemäß dem Grad des optimalen Schnittabschnittes zu sortieren (225);
- ein Auswahlsystem, das konfiguriert ist, um eine Charge von sortierten rohen Holzschichten gemäß dem Grad des optimalen Schnittabschnittes auszuwählen;
- ein Beschneidungssystem (241), das konfiguriert ist, um die Länge der rohen Holzschicht gemäß der Länge des optimalen Schnittabschnittes zu beschneiden; und
- ein Stutzsystem (242), das konfiguriert ist, um die Breite der rohen Holzschicht gemäß der Breite des optimalen Schnittabschnittes zu stutzen.

11. Datenverarbeitungssystem, das konfiguriert ist, um das computerimplementierte Verfahren (300) nach einem der Ansprüche 1 bis 8 durchzuführen.

12. Computerprogramm, umfassend Anweisungen, die, wenn das Programm durch einen Computer ausgeführt wird, bewirken, dass der Computer das computerimplementierte Verfahren (300) nach einem der Ansprüche 1 bis 8 durchführt.

13. Computerlesbares Medium, das Anweisungen umfasst, die, wenn sie durch einen Computer ausgeführt werden, bewirken, dass der Computer das computerimplementierte Verfahren (300) nach einem der Ansprüche 1 bis 8 durchführt.

## Revendications

1. Procédé mis en œuvre par ordinateur (300) permettant le traitement d'une couche de bois brut pour la fabrication de panneaux de bois décoratifs, le procédé comprenant les étapes suivantes :
- l'obtention (310) d'une image numérique de la couche de bois brut ;
- la détection (320) de caractéristiques de détermination de qualité (324) de la couche de bois brut à partir de l'image numérique ;
ledit procédé mis en œuvre par ordinateur étant **caractérisé par**
- la détermination (330) d'une section de coupe candidate sur la couche de bois brut, ladite section de coupe candidate définissant un recadrage de la couche de bois brut en une forme rectangulaire (402) comportant une longueur minimale qui est déterminée sur la base d'une longueur des panneaux de bois décoratifs ;
- la détermination (350) d'une note de la section de coupe candidate à partir des caractéristiques de détermination de qualité comprises dans ladite section de coupe ;
- la détermination (360) d'un rendement de la section de coupe candidate sur la base d'une surface (344) de la section de coupe candidate et de la note (351) ; et
- la détermination (370) d'une section de coupe optimale en tant que section de coupe candidate qui aboutit à un rendement le plus élevé en effectuant, de manière itérative :
- le réglage d'une longueur, d'une largeur et/ou d'un emplacement de la section de coupe candidate (402) sur la couche de bois brut (401) ; et
- la détermination du rendement pour la section de coupe candidate réglée (330, 350, 360).

2. Procédé mis en œuvre par ordinateur selon la revendication 1, ladite détection (320) des caractéristiques de détermination de qualité étant réalisée par un premier classificateur (520) formé pour détecter lesdites caractéristiques de détermination de qualité (530) à partir de l'image numérique (510).

3. Procédé mis en œuvre par ordinateur selon l'une quelconque des revendications précédentes, lesdites caractéristiques de détermination de qualité (324) caractérisant un type de caractéristique sélectionné dans le groupe comprenant au moins un parmi un défaut (321), une couleur (322) et un motif de coupe (323).

4. Procédé mis en œuvre par ordinateur selon la revendication 3, une caractéristique de détermination de qualité du type de défaut (321) étant sélectionnée dans le groupe comprenant au moins un parmi un nœud, un trou de nœud, une ligne minérale, une piqûre, une tache, une loupe, une erreur de coupe, un grain grossier, un grain surélevé, un aubier, un dommage fongique, un défaut d'insecte, une fissure, une fente et une fibre torsadée.

5. Procédé mis en œuvre par ordinateur selon la revendication 3, une caractéristique de détermination de qualité du type de motif de coupe (323) étant sélectionnée dans le groupe comprenant au moins une parmi une coupe rotative, une coupe en couronne, une coupe en quart de normale, une coupe en quart de fente ou une combinaison de celles-ci.

6. Procédé mis en œuvre par ordinateur selon la revendication 3, une caractéristique de détermination de qualité du type de couleur (322) étant sélectionnée dans le groupe comprenant au moins une parmi une couleur primaire, une couleur secondaire, une distribution de couleur, une différence de couleur et une variation de couleur.

7. Procédé mis en œuvre par ordinateur selon l'une quelconque des revendications précédentes, ladite détermination (350) de la note de la section de coupe candidate étant réalisée par un second classificateur (620), formé pour déterminer ladite note (630) à partir des caractéristiques de détermination de qualité (610) comprises dans ladite section de coupe candidate.

8. Procédé mis en œuvre par ordinateur selon la revendication 7, comprenant en outre l'obtention d'au moins une propriété de production de la couche de bois brut, et ledit second classificateur (620) étant en outre formé pour déterminer ladite note (630) à partir d'une ou plusieurs propriétés de production.

9. Procédé (200) permettant le traitement d'une couche de bois brut (130), comprenant :
- l'obtention de la couche de bois brut (130) ;
- la capture d'une image numérique de la couche de bois brut au moyen d'une caméra (221) ;
- par une unité de commande (223), la détermination d'une section de coupe optimale avec un rendement le plus élevé à partir de ladite image numérique en réalisant le procédé mis en œuvre par ordinateur selon l'une quelconque des revendications 1 à 8 ;
- le tri (225) de la couche de bois brut selon la note de la section de coupe optimale ;
- la prise d'un lot de couches de bois brut triées selon la note de la section de coupe optimale ;
- le taillage (241) de la longueur de la couche de bois brut selon la longueur de la section de coupe optimale ; et
- le massicotage (242) de la largeur de la couche de bois brut selon la largeur de la section de coupe optimale.

10. Système de traitement d'une couche de bois brut, le système comprenant :
- une caméra (221) configurée pour obtenir une image numérique de la couche de bois brut (130) ;
- une unité de commande (223) configurée pour déterminer une section de coupe optimale avec un rendement le plus élevé à partir de ladite image numérique en réalisant le procédé mis en œuvre par ordinateur selon l'une quelconque des revendications 1 à 8 ;
- un système de tri (224) configuré pour trier (225) la couche de bois brut selon la note de la section de coupe optimale ;
- un système de prise configuré pour prendre un lot de couches de bois brut triées selon la note de la section de coupe optimale ;
- un système de taillage (241) configuré pour tailler la longueur de la couche de bois brut selon la longueur de la section de coupe optimale ; et
- un système de massicotage (242) configuré pour massicoter la largeur de la couche de bois brut selon la largeur de la section de coupe optimale.

11. Système de traitement de données configuré pour réaliser le procédé mis en œuvre par ordinateur (300) selon l'une quelconque des revendications 1 à 8.

12. Programme informatique comprenant des instructions qui, lorsque le programme est exécuté par un ordinateur, amènent l'ordinateur à réaliser le procédé mis en œuvre par ordinateur (300) selon l'une quelconque des revendications 1 à 8.

13. Support lisible par ordinateur comprenant des instructions qui, lorsqu'elles sont exécutées par un ordinateur, amènent l'ordinateur à réaliser le procédé mis en œuvre par ordinateur (300) selon l'une quelconque des revendications 1 à 8.
